**Europäisches Patentamt**

**European Patent Office**

**Office européen des brevets**

(19)

(11) Numéro de publication: **0 131 501**
**B1**

(12) **FASCICULE DE BREVET EUROPEEN**

(45) Date de publication du fascicule du brevet:
**17.02.88**

(21) Numéro de dépôt: **84401317.7**

(22) Date de dépôt: **22.06.84**

(51) Int. Cl.⁴: **A 61 M 11/00, B 65 D 83/14**

(54) Embout-poussoir pour pulvérisateur médical.

(30) Priorité: **24.06.83 FR 8310450**

(43) Date de publication de la demande:
**16.01.85 Bulletin 85/3**

(45) Mention de la délivrance du brevet:
**17.02.88 Bulletin 88/7**

(84) Etats contractants désignés:
**AT BE CH DE FR GB IT LI LU NL SE**

(56) Documents cités:
**DE - B - 1 274 283**
**US - A - 3 471 092**
**US - A - 3 482 784**
**US - A - 3 961 756**
**US - A - 4 029 261**

(73) Titulaire: **ETABLISSEMENTS VALOIS Société Anonyme dite:, Boîte Postale G Le Prieuré, F-27110 Le Neubourg (FR)**

(72) Inventeur: **Brunet, Michel, 7 rue des Pensées, F-27840 Ste Colombe la Commanderie (FR)**
Inventeur: **Garcia, Firmin, 41 Avenue A. Briand, F-27000 Evreux (FR)**

(74) Mandataire: **Pinguet, André, CAPRI 28 bis, avenue Mozart, F-75016 Paris (FR)**

ACTORUM AG

# Description

La présente invention a pour objet un embout formant poussoir pour pulvérisateur médical, destiné à pulvériser des médicaments ou autres produits d'hygiène dans les narines, les oreilles, la bouche et autres orifices.

L'invention concerne aussi bien les dispositifs de pulvérisation, doseurs ou non, montés sur un bidon sous pression que les pompes actionnées à la main (pression d'un doigt).

L'invention vise plus particulièrement les buses ou pulvérisateurs comportant un organe indépendant, destiné à imprimer au produit un mouvement tourbillonnaire et dispersant, appelé communément gicleur. Il existe des poussoirs, qui peuvent être montés sur la tige de soupape d'une valve, qui ne comportent aucune pièce rapportée. Le mouvement tourbillonnaire du produit projeté est provoqué par des creux et des reliefs, formés sur la surface interne du poussoir et/ou sur l'extrémité de la tige de soupape. L'inconvénient de ces systèmes, est leur prix de revient peu compétitif et l'inadaptabilité d'un poussoir quelconque sur n'importe quelle soupape (voir Brevet FR-A-1 319 576).

Les dispositifs les plus répandus comportent un poussoir, qui s'engage exactement sur une tige de soupape, qui est absolument standard. Le poussoir comporte un canal interne qui prolonge le canal central de la tige de soupape. Le canal du poussoir se termine à l'extérieur par une cavité élargie dans laquelle est logé le gicleur. Le gicleur est maintenu par serrage, étant enfoncé à force, et/ou par encliquetage au moyen de nervures appropriées ce système a l'avantage de permettre le montage de n'importe quel bouton-poussoir, sur n'importe quelle tige de soupape, et ce dans des conditions de prix de revient avantageuses.

La tenue du gicleur dans son logement est en général bonne et il est assez rare que le gicleur soit poussé hors de son logement par la pression du fluide cherchant à s'échapper. Si un accident de ce genre est sans gravité quand il s'agit de projeter un parfum ou même un insecticide, il y a des cas où un tel risque, même minime, ne peut pas être accepté. Cela limite les applications des aérosols.

Il est connu de l'art antérieur (US-A 3 471 092) de réaliser un poussoir pour pulvérisateur aérosol dont aucun organe ne puisse être accidentellement expulsé en cours de fonctionnement. Un tel poussoir est constitué par une pièce extérieure comportant un canal central longitudinal et par une pièce intérieure de forme sensiblement allongée et logée dans ledit canal. Le canal longitudinal est partiellement fermé à sa partie avant par une paroi d'extrémité percée d'un orifice de pulvérisation et comporte à sa partie arrière une extrémité ouverte enfichable sur la tige de soupape d'une valve d'un récipient sous pression, cette extrémité ouverte étant pourvue d'un épaulement limitant l'enfoncement de ladite tige de soupape dans ledit canal. La pièce extérieure et la pièce intérieure délimitent à leur partie avant une chambre tourbillonnaire placée en amont de l'orifice de pulvérisation et latéralement au moins un passage intérieur longitudinal pour servir à mettre en communication ladite chambre tourbillonnaire l'orifice de la tige de soupape.

S'ils présentent toutes les garanties de sécurité requises d'un appareil à destination médicale, les poussoirs de ce type existant présentent cependant cet inconvénient que, d'une part, leur pièce intérieure est de forme complexe et nécessite donc d'être moulée et que, d'autre part, cette même pièce intérieure n'occupe qu'un volume réduit dans le canal intérieur, ce qui autorise l'accumulation et la stagnation de produit dans le poussoir lui-même.

La présente invention a pour objet un poussoir pour pulvérisateur aérosol de forme allongée pour permettre son introduction dans un orifice buccal, comportant une pièce extérieure et une pièce intérieure comme la réalisation de l'art antérieur précitée, mais n'en présentant pas les inconvénients, ledit poussoir étant caractérisé en ce que sa pièce intérieure est constituée par une tige de section essentiellement constante sur toute sa longueur et de dimensions sensiblement égales à celles du volume du canal longitudinal, ladite pièce intérieure comportant en outre un méplat longitudinal délimitant avec la paroi latérale du canal de sa pièce extérieure un passage longitudinal et comportant au moins à sa partie avant une face d'extrémité perpendiculaire à l'axe de la tige, ladite face d'extrémité avant de la pièce intérieure coopérant avec la paroi d'extrémité avant dudit canal pour former une chambre tourbillonnaire, la face d'extrémité arrière de la pièce intérieure adjacente à la tige de soupape d'une valve d'un récipient sous pression étant espacée de ladite tige de soupape de manière à délimiter avec cette dernière et avec la surface intérieure du canal longitudinal un volume mort communiquant avec ledit passage longitudinal, ladite paroi d'extrémité avant du canal longitudinal comportant au moins une rainure non radiale pour mettre en communication le passage longitudinal et la chambre tourbillonnaire.

D'autres caractéristiques de l'invention apparaîtront au cours de la description qui va suivre, donnée à titre d'exemple non limitatif en regards des dessins cijoints, et qui fera bien comprendre comment l'invention peut être réalisée.

Sur les dessins,

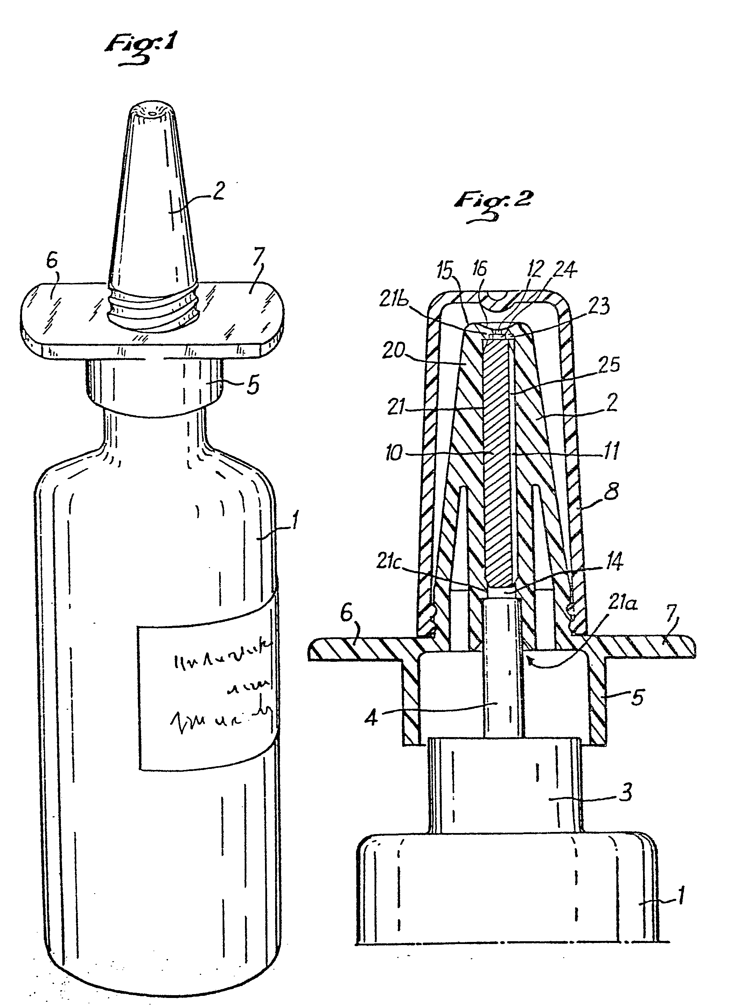

– la figure 1 est une vue en perspective d'un flacon aérosol équipé d'un embout nasal selon l'invention; et
– la figure 2 est une coupe axiale de l'embout de la figure 1;

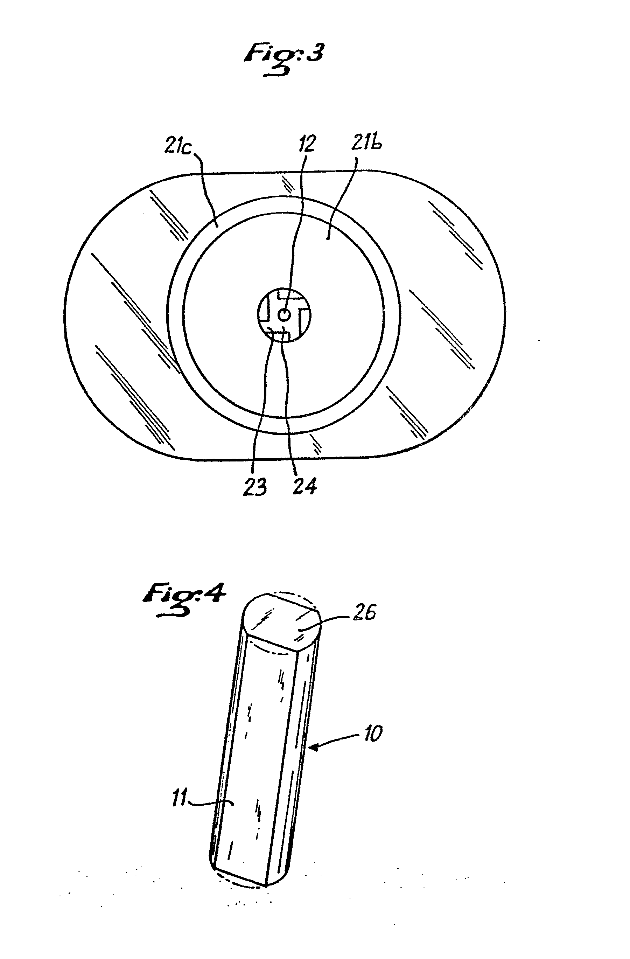

– la figure 3 est une vue axiale de l'embout dans le sens de la flèche sur la figure 2; et
– la figure 4 représente en perspective, un des éléments du poussoir selon l'invention.

Le flacon 1 est équipé de l'embout nasal 2. Le flacon peut être souple, la pression étant fournie par serrage à la main ou être résistant et comporter un gaz sous pression, dissout ou non dans le liquide à pulvériser. Le système de pulvérisation peut aussi être une pompe actionnée à la main. La valve 3, quel que soit son principe, présente une tige de soupape 4 classique sur laquelle peut être monté n'importe quel bouton poussoir standard.

Le poussoir 2 est de forme cônique allongée, pour pouvoir pénétrer dans les narines ou l'oreille d'un enfant, par exemple pour y pulvériser un médicament ou tout autre liquide d'hygiène. Il est bien entendu qu'il s'agit d'un exemple non limitatif. Le poussoir comporte une jupe 5 masquant la capsule et deux ailes 6, 7 permettant une bonne prise, ou permettant l'actionnement plus facile. L'ensemble est complété par un capuchon 8 encliquetable.

Conformément à l'invention, l'embout poussoir comporte une première pièce extérieure 20 qui est formé avec un canal central axial 21 cylindrique, ayant une extrémité ouverte 21a et une extrémité délimitée par une paroi 21b. L'ouverture 21a est adaptée à être montée à frottement sur la tige de soupape 4. Le canal 21 comporte à cet effet un épaulement 21c permettant de limiter l'enfoncement de la tige de soupape dans le canal.

La paroi 21b comporte un orifice 12, de dimension inférieure à la section du canal. C'est l'orifice de pulvérisation. Sur la face interne de la paroi 21b, en regard du canal 21, sont formées des rainures 23 s'étendant depuis la paroi cylindrique du canal 21, jusque dans une cavité placée en amont de l'orifice 12, formant une chambre tourbillonnaire 24. Les rainures 23 (il en faut au moins une, il peut y en avoir plusieurs, quatre par exemple) ne sont pas radiales, de façon à imprimer au produit qui sort par l'orifice 12 un mouvement tourbillonnaire pour favoriser une bonne pulvérisation.

Le volume du canal 21 est rempli sur une partie de la section, et sur la plus grande partie de sa longueur par une tige 10, par exemple de section ronde, de diamètre égal à celui du canal, et avec un ou deux méplats 11. Le but de la tige 10 est de limiter la section de débit du produit, et de réduire le volume mort 14 entre la valve 3 et le gicleur formé par la chambre 24 et les rainures 23, coopérant avec la surface d'extrémité 26 de la tige 10. On pourrait aussi utiliser par exemple une tige de section carrée inscrite dans la section du canal. Il reste donc un ou plusieurs passages longitudinaux 25, amenant le produit, chassé par la valve 3, à la périphérie de la paroi 21b. Le produit est chassé par les rainures 23, la chambre 24 et l'orifice 12, ce qui réalise une excellente pulvérisation.

Grâce à la présente invention, on réalise donc le poussoir, l'embout nasal et de gicleur, avec seulement deux pièces, dont seulement une doit être moulée. La tige 10 est en effet simplement coupée à la longueur.

Cette construction permet de réaliser l'embout avec des surfaces arrondies 15, 16 directement de moulage, sans arêtes vives ou anguleuses que l'on rencontre en général sur le bord du logement du gicleur quand il est extérieur, et qui risqueraient de blesser les muqueuses de l'utilisateur. Le volume mort 14, très réduit, limite les pertes de produit et les risques de pollution de produit pouvant séjourner hors du flacon dans des conditions d'hygiène non contrôlées. Le gicleur ne risque pas d'être éjecté par la pression du produit. L'éjection du gicleur, dans une oreille ou dans les fosses nasales ou les sinus ou dans la gorge d'un utilisateur ou tout autre orifice corporel, pourrait en effet avoir des conséquences très graves, toujours désagréables.

Il va de soi que le mode de réalisation décrit n'est qu'un exemple et qu'il serait possible de le modifier, notamment par substitution d'équivalents techniques, sans sortir pour cela du cadre de l'invention.

**Revendication**

Poussoir pour pulvérisateur aérosol de forme allongée pour permettre son introduction dans un orifice corporel, ledit poussoir étant constitué par une pièce extérieure (20) comportant un canal central longitudinal (21) et par une pièce intérieure (10) de forme sensiblement allongée et logée dans ledit canal, ledit canal longitudinal (21) étant partiellement fermé à sa partie avant par une paroi d'extrémité (21b) percée d'un orifice de pulvérisation (12) et comportant à sa partie arrière une extrémité ouverte (21a) enfichable sur la tige de soupape (4) d'une valve (3) d'un récipient sous pression, ladite extrémité ouverte (21a) étant pourvue d'un épaulement (21c) limitant l'enfoncement de ladite tige de soupape dans ledit canal, ces deux pièces (10, 20) délimitant à leur partie avant une chambre tourbillonnaire (24) placée en amont de l'orifice de pulvérisation (12) et latéralement au moins un passage intérieur longitudinal (25) pour servir à mettre en communication ladite chambre tourbillonnaire (24) et l'orifice de la tige de soupape (4), ledit poussoir étant caractérisé en ce que: ladite pièce intérieure (10) est constituée par une tige de section essentiellement constante sur toute sa longueur et de dimensions sensiblement égales à celles du volume du canal longitudinal (21), ladite pièce intérieure comportant en outre un méplat longitudinal (11) délimitant avec la paroi latérale dudit canal (21) ledit passage longitudinal (25) et comportant au moins à sa partie avant une face d'extrémité (26) perpendiculaire à l'axe de la tige, ladite face d'extrémité avant (26) de la pièce intérieure coopérant avec la paroi d'extrémité avant (21b) dudit canal pour former la chambre tourbillonnaire (24), la face d'extrémité arrière de la pièce intérieure (10) adjacente à la tige de soupape (4) étant espacée de celle-ci de manière à délimiter avec cette dernière et avec la surface intérieure du canal longitudinal un volume mort (14) communiquant avec ledit passage longitudinal (25), ladite paroi d'extrémité avant (21b) du canal longitudinal comportant au moins une rainure (23) non radiale pour mettre en communication le passage (25) et la chambre tourbillonnaire (24).

## Claim

Plunger for an aerosol atomiser, of elongated shape to allow its insertion into a bodily orifice, the said plunger comprising an outer member (20) having a central longitudinal channel (21) and an inner member (10) of substantially elogated form and housed in the said channel, the said longitudinal channel (21) being partially closed at its forward portion by an end wall (21b) pierced by an atomising orifice (12) and at its rear portion comprising an open extremity (21a) which may be pushed on to the valve stem (4) of a valve (3) of a container under pressure, the said open extremity (21a) being provided with a shoulder (21c) limiting the insertion of the said valve stem into the said passage, these two members (10, 20) in their front portion delimiting a turbulence chamber (24) positioned upstream from the atomising orifice (12) and laterally at least one longitudinal internal passage (25) acting to establish communication between the said turbulence chamber (24) and the orifice of the valve stem (4), the said plunger being characterised in that: the said inner member (10) is formed by a rod of substantially constant cross-section throughout its length and of dimensions substantially equal to those of the volume of the longitudinal channel (21), the said inner member furthermore comprising a longitudinal flat (11) which with the sidewall of the said channel (21) delimits the said longitudinal passage (25) and at least at its forward portion comprising an end surface (26) at right angles to the axis of the stem, the said front end surface (26) of the inner member cooperating with the forward end surface (21b) of the said channel to form the turbulence chamber (24), the rear end surface of the inner member (10) adjacent to the valve stem (4) being spaced apart from the same in such manner that, with this latter and with the internal surface of the longitudinal channel, it delimits an idle volume (14) in communication with the said longitudinal passage (25), the said front end surface (21b) of the longitudinal passage comprising at least one non-radial groove (23) to establish communication between the passage (25) and the turbulence chamber (24).

## Patentanspruch

Drücker für einen langgestreckten Aerosol-Zerstäuber für die Einführung in eine Körperöffnung, wobei der Drücker besteht aus einem äusseren Teil (20) mit einem zentralen Längskanal (21) und einem inneren Teil (10) mit im wesentlichen langgestreckter Form, das in dem Kanal angeordnet ist, wobei der Längskanal (21) an seinem vorderen Ende durch eine Stirnwand (21b) mit einer Zerstäubungsöffnung (12) teilweise verschlossen ist und an seinem hinteren Abschnitt ein offenes Ende (21a) aufweist, das auf den Stössel (4) eines Ventils (3) eines unter Druck stehenden Behälters aufsteckbar ist, wobei das offene Ende (21a) mit einem Schulterstück (21c) ausgestattet ist, welches das Hineindrücken des Ventilstössels in den Kanal begrenzt, wobei diese beiden Teile (10, 20) an ihrem vorderen Abschnitt eine oberhalb der Zerstäubungsöffnung (12) angeordnete Verwirbelungskammer (24) und seitlich mindestens einen inneren Längskanal (25), der die Verwirbelungskammer (24) mit der Öffnung des Ventilstössels (4) verbindet, begrenzen, wobei der Drücker dadurch gekennzeichnet ist, dass der innere Teil (10) besteht aus einem Schaft, der über seine gesamte Länge einen im wesentlichen konstanten Querschnitt hat und Dimensionen aufweist, die im wesentlichen gleich denjenigen des Volumens des Längskanals (21) sind, wobei der innere Teil ausserdem eine langgestreckte Zwischenplatte (11), die zusammen mit der Seitenwand des Kanals (21) den Längskanal (25) begrenzt, und mindestens an seinem vorderen Abschnitt eine Stirnfläche (26) senkrecht zur Achse des Ventilstössels aufweist, wobei die vordere Stirnfläche (26) des inneren Teils mit der vorderen Stirnwand (21b) des Kanals kooperiert zur Bildung der Verwirbelungskammer (24), wobei die hintere Stirnfläche des inneren Teils (10), die an den Ventilstössel (4) angrenzt, einen Abstand von diesem hat, so dass sie zusammen mit diesem und mit der inneren Oberfläche des Längskanals ein Totvolumen (14) begrenzt, das eine Verbindung zu dem Längskanal (25) herstellt, und die vordere Stirnwand (21b) des Längskanals mindestens eine nicht-radiale Nut (23) aufweist, um den Kanal (25) mit der Verwirbelungskammer (24) zu verbinden.

_Fig:1_

_Fig:2_

## Fig.3

## Fig.4